# EUROPÄISCHE PATENTSCHRIFT

(11) **EP 0 845 503 B1**
(45) Veröffentlichungstag und Bekanntmachung des Hinweises auf die Patenterteilung: **06.06.2001**
(21) Anmeldenummer: 97119150.7
(22) Anmeldetag: 03.11.1997
(51) Int. Cl.: C09B 61/00, A23L 1/275, A23K 1/16, A61K 7/48, A61K 9/107

(54) **Flüssige, mit Öl mischbare Carotinoid-Zubereitungen**
Liquid carotenoid compound preparations miscible with oil
Compositions de caroténoides liquides miscibles à l'huile

(30) Priorität: 27.11.1996 DE 19649062
(43) Veröffentlichungstag der Anmeldung: 03.06.1998
(73) Patentinhaber: BASF AKTIENGESELLSCHAFT, 67056 Ludwigshafen (DE)
(72) Erfinder: Lüddecke, Erik, Dr., 67112 Mutterstadt (DE); Pfeiffer, Angelika-Maria, Dr., 68165 Mannheim (DE); Meyer, Joachim, Dr., 67133 Maxdorf (DE)

(56) Entgegenhaltungen:
- EP-A- 0 065 193
- EP-A- 0 441 495
- WO-A-92/07477
- DE-B- 1 211 911
- FR-A- 2 281 961
- GB-A- 993 138

## Beschreibung

Die Erfindung betrifft flüssige, mit Öl mischbare Carotinoid-Zubereitungen, Verfahren zu deren Herstellung sowie ihre Verwendung als Zusatz zu Tierfuttermitteln, Lebensmitteln, Kosmetika und Pharmazeutika.

Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, Astaxanthin, β-Apo-8'-carotinal, Canthaxanthin, Lycopin und Citranaxanthin genannt. Sowohl für die Lebensmittel- und Futtermittelindustrie als auch für die pharmazeutische Technologie stellen diese synthetisch herstellbaren Substanzen z.B. als Ersatz für künstliche Farbstoffe wichtige Farbkörper dar und sind zum Teil wegen ihrer Pro-Vitamin-A-Aktivität von Interesse.

Alle Carotinoide sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlecht resorbiert werden und somit nur schlechte Färbungsergebnisse liefern.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Carotinoide und damit indirekt zu besseren Färbungseffekten z.B. bei der Eidotter- oder Fischpigmentierung.

In der Futtermittelindustrie, speziell im Bereich Fishfarming werden aus der Gruppe der Carotinoide besonders Astaxanthin und Canthaxanthin zum Färben von u.a. Lachsen, Forellen und Shrimps verwendet. Dabei werden die Wirkstoffe in Form von Trockenpulvern eingesetzt, in denen das dispergierte Carotinoid durch z.B. Gelatine und Zucker als Schutzkolloide vor oxidativem Abbau geschützt wird. Das Trockenpulver wird dabei während der Futtermittelherstellung direkt im Extrusions- bzw. Pelletierverfahren mit verarbeitet, wobei die unter den Herstellbedingungen auftretenden Streßfaktoren wie Hitze, Feuchtigkeit und Lufteinfluß häufig zu unerwünschten Produktschädigungen und damit zu Wirkstoffverlusten führen.

Zur Verringerung dieser Wirkstoffverluste kann, wie in WO 96/23420 beschrieben, feingemahlenes Astaxanthin in Öl suspendiert werden, um anschließend auf das bereits extrudierte bzw. pelletierte Futtermittel aufgesprüht zu werden. Sowohl die Bioverfügbarkeit und damit auch die erzielte Farbwirkung als auch die chemische Stabilität der Wirkstoffe sind hierbei nicht zufriedenstellend.

In anderen Verfahren, beschrieben in WO 94/19411, wird kristallines β-Carotin in Gegenwart einer wäßrigen Schutzkolloid-Lösung gemahlen und anschließend durch kurzzeitiges Erhitzen bis zum Schmelzpunkt in eine amorphe Modifikation überführt.

Auch diese Formulierung, sowie die zahlreich beschriebenen, wäßrigen Carotinoid-Dispersionen und O/W-Emulsionen, in denen der Wirkstoff in Gegenwart stabilisierender Schutzkolloide vorliegt, sind ebenfalls ungeeignet, da sie mit Ölen nicht mischbar sind.

GB 993 138 beschreibt ein Verfahren zur Herstellung von u.a. Vitamin A-haltigen Beadlets, das dadurch gekennzeichnet ist, daß man Vitamin A in eine wäßrige, Gelatine-haltige Schutzkolloid-Lösung dispergiert und diese Dispersion durch zusätzliches Suspendieren in Öl in Form von Beadlets ausfällt. Aufgrund von zu großen Partikeldurchmessern von über 300 µm und der damit verbundenen physikalischen Instabilität solcher Suspensionen bzw. Emulsionen, sind solche Systeme für die oben genannte Anwendung von flüssigen Carotinoiden-Zubereitungen ungeeignet.

Es bestand daher die Aufgabe, Carotinoid-Formulierungen zu entwickeln, die die oben genannten Nachteile nicht aufweisen.

Diese Aufgabe wurde erfindungsgemäß mit der Bereitstellung flüssiger, mit Öl mischbarer Carotinoid-Zubereitungen gelöst, die dadurch gekennzeichnet sind, daß sie als doppelte Dispersionssysteme eine wäßrig-disperse Phase mit einem Partikeldurchmesser kleiner 100 µm, in der schutzkolloid-stabilisierte Teilchen eines oder mehrerer Carotinoide dispergiert vorliegen, in einem Öl als Dispersionsmittel enthalten.

Die Erfindung betrifft außerdem ein Verfahren zur Herstellung von flüssigen, mit Öl mischbaren Carotinoid-Zubereitungen dadurch gekennzeichnet, daß man eine wäßrige, schutzkolloidhaltige Dispersion eines oder mehrerer Carotinoide in Gegenwart eines Emulgators in Öl emulgiert.

Durch die erfindungsgemäße Kombination wäßriger Dispergierverfahren von fettlöslichen Wirkstoffen mit einem weiteren Wasser-in-Öl-Emulgierschritt lassen sich Produkte mit neuen überraschenden Eigenschaftsprofilen erhalten.

Die so erhältlichen flüssigen Carotinoid-Zubereitungen lassen sich hervorragend als Farbstoffe bzw. Zusätze für Lebens- und/ oder Futtermittel sowie für kosmetische oder pharmazeutische Darreichungsformen verwenden.

Die Herstellung der wäßrig-dispersen Phase, die schutzkolloid-stabilisierte Teilchen eines oder mehrerer Carotinoide enthält, ist bereits Gegenstand zahlreicher Publikationen und Patentschriften, wie beispielsweise EP-B-0 278 284, EP-B-0 065 193, DE-A-3 702 030, DE-A-2 534 091 sowie einer Zusammenfassung in R.A. Morton, Fat Soluble Vitamins, Intern. Encyclopedia of Food and Nutrition, Bd. 9, Pergamon Press 1970, S. 134-139.

Aus der Vielzahl der oben zitierten, wäßrigen Dispergierverfahren ist das sogenannte Mikronisierverfahren gemäß EP-B-0 065 193 für die vorliegenden Zwecke besonders geeignet. Dieses Verfahren ist dadurch gekennzeichnet, daß man ein Carotinoid in einem flüchtigen, mit Wasser mischbaren, organischen Lösungsmittel bei Temperaturen zwischen 50°C und 200°C innerhalb einer Zeit von weniger als 10 Sekunden löst und aus der erhaltenen Lösung sofort durch schnelles Mischen mit einer wäßrigen Schutzkolloid-Lösung das Carotinoid in kolloid-disperser Form ausfällt. Bezüglich der Einzelheiten wird auf die EP-B-0 065 193 verwiesen.

Die Carotinoide, die im Rahmen der Erfindung eingesetzt werden können, sind die bekannten, zugänglichen, natürlichen oder synthetischen Vertreter dieser Klasse von Verbindungen, die als farbgebende Mittel brauchbar sind, z.B. β-Carotin, Astaxanthin, Lycopin, Bixin, Zeaxanthin, Cryptoxanthin, Citranaxanthin, Lutein, Canthaxanthin, β-Apo-4'-carotinal, β-Apo-8'-carotinal, β-Apo-12'-carotinal, β-Apo-8'-carotinsäure sowie Ester von hydroxy-und carboxyhaltigen Vertretern dieser Gruppe, z.B. die niederen Alkylester und vorzugsweise die Methyl- und Ethylester. Besonders bevorzugt werden die bisher technisch gut zugänglichen Carotinoide wie β-Carotin, Astaxanthin, Canthaxanthin, Lycopin, β-Apo-8'-carotinal und β-Apo-8'-carotinsäureester einzeln oder als Mischung verwendet. Die Wirkstoffe liegen dabei in öliger, amorpher und/oder kristalliner Form vor, wobei im Hinblick auf eine optimale Bioverfügbarkeit die öligen und amorphen Modifikationen bevorzugt sind.

Nach dem o.g. Mikronisierverfahren sind zur Durchführung der ersten Dispergierstufe als Teil des Gesamtverfahrens vor allem wassermischbare, thermisch stabile, flüchtige, nur Kohlenstoff, Wasserstoff und Sauerstoff enthaltene Lösungsmittel wie Alkohole, Ether, Ester, Ketone und Acetale geeignet. Vorzugsweise werden Ethanol, n-Propanol, Isopropanol, 1,2-Butandiol-1-methylether, 1,2-Propandiol-1-n-propylether oder Aceton verwendet. Allgemein verwendet man zweckmäßig solche Lösungsmittel, die mindestens zu 10% wassermischbar sind, einen Siedepunkt unter 200°C aufweisen und/oder weniger als 10 Kohlenstoffe haben.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Die Mengen an Schutzkolloid, Weichmacher und Carotinoid werden im allgemeinen so gewählt, daß ein Endprodukt erhalten wird, das zwischen 0,01 und 10 Gew.-%, vorzugsweise zwischen 0,5 und 5 Gew.-%, Carotinoid, 2 bis 20 Gew.-% eines Schutzkolloids, 2 bis 20 Gew.-% eines Weichmachers, alle Prozentangaben bezogen auf die Gesamtmasse der fertigen Emulsion, sowie gegebenenfalls geringe Mengen eines Stabilisators enthält.

Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol, Ascorbinsäure oder Ethoxyquin zuzusetzen. Sie können entweder der wäßrigen oder der Lösungsmittel-Phase zugesetzt werden, vorzugsweise werden sie jedoch gemeinsam mit den Farbstoffen und gegebenenfalls zusätzlichen Emulgatoren in der Lösungsmittel-Phase gelöst.

Als Emulgatoren in der primären Dispersion können beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 10 bis 150 Gew.%, besonders bevorzugt 15 bis 80 Gew.%, bezogen auf das/die Carotinoid(e), verwendet werden.

Durch Zusatz von in der Lebens- und Futtermittelindustrie zugelassenen Konservierungsstoffen wie beispielsweise Sorbinsäure, Natrium-Sorbat, Benzoesäure, Natrium-Benzoat oder PHB-Ester wie 4-Hydroxy-methylbenzoat oder 4-Hydroxy-propylbenzoat in einer Konzentration von 0 bis 200 Gew.%, vorzugsweise 10 bis 150 Gew.%, besonders bevorzugt 15 bis 80 Gew.%, bezogen auf das/die Carotinoid(e), kann eine unerwünschte mikrobielle Zersetzung der wäßrigen Dispersion verhindert werden.

Unter Umständen kann es auch vorteilhaft sein, zusätzlich zum Carotinoid oder dem Gemisch mehrerer Carotinoide im ersten Dispergierschritt ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf das/die Carotinoid(e), zu lösen, das dann gemeinsam mit den Wirkstoffen und den genannten Zusatzstoffen beim Mischen mit der wässrigen Phase extrem feinteilig ausgefällt wird.

Je nach Art und Menge des verwendeten Schutzkolloids erhält man als wäßrige Carotinoid-Dispersion eine tiefgefärbte viskose Flüssigkeit, die im Falle eines gelierfähigen Kolloids gelartig erstarrt und in der die mittlere Teilchengröße der Carotinoide weniger als 0,2 µm beträgt.

Die Entfernung des Lösungsmittels kann je nach Siedepunkt in an sich bekannter Weise, z.B. durch Destillation, gegebenenfalls unter vermindertem Druck, oder durch Extraktion mit einem mit Wasser-nicht mischbaren Lösungsmittel, erfolgen.

Anders als im oben erwähnten Stand der Technik, wonach die wäßrigen Wirkstoff-Dispersionen in ein Trockenpulver überführt werden, führt im erfindungsgemäßen Verfahren die zusätzliche Wasser-in-Öl-Emulgierung zu einer neuen Flüssigformulierung in Form einer doppelten Dispersion.

Dabei wird unter Verwendung eines Emulgators eine Wasser-in-Öl-Emulsion gebildet, in der die Wasserphase schutzkolloidstabilisierte Carotinoid-Nanoteilchen enthält. Als Emulgatoren kommen an sich bekannte W/O-Emulgatoren mit einem HLB-Wert kleiner 10, insbesondere von 2 bis 6 in Betracht (vgl. H.P. Fiedler, Lexikon der Hilfsstoffe für Pharmazie, Kosmetik und angrenzende Gebiete, 1996, Seiten 753 ff). Typische Vertreter dieser Emulgatorklasse sind Partialfettsäureester mehrwertiger Alkohole z.B. Glycerolmonostearat oder Mischungen aus Mono-, Di- und Triglyceriden, Partialfettsäureester des Sorbitans und/oder bevorzugt Fettsäureester des Polyglycerols wie beispielsweise Polyglycerol Polyricinoleat, die in einer Konzentration von 10 bis 1000 Gew.%, vorzugsweise 100 bis 900 Gew.%, besonders bevorzugt 400 bis 800 Gew.%, bezogen auf das/die Carotinoid(e), verwendet werden.

Das Dispersionsmittel kann sowohl mineralischen, pflanzlichen als auch tierischen Ursprungs sein. Typische Vertreter sind eßbare Öle, insbesondere Sesamöl, Maiskeimöl, Baumwollsaatöl, Sojabohnenöl oder Erdnußöl, Ester mittelkettiger pflanzlicher Fettsäuren sowie unterschiedliche Fischöle wie beispielsweise Makrelen-, Sprotten- oder Lachsöl. Die Menge des Dispersionsmittels beträgt im allgemeinen 30 bis 95, vorzugsweise 50 bis 80 Gew.%, bezogen auf die Gesamtmasse der fertigen Emulsion.

Man kann das Emulgieren kontinuierlich oder diskontinuierlich durchführen.

Die physikalische Stabilität des doppelten Dispersionssystems, wie etwa die Sedimentationsstabilität, wird erreicht durch eine sehr gute Feinverteilung der Wasserphase in der Ölphase, z.B. durch intensive Behandlung mit einem Rotor/Stator-Dispergator bei Temperaturen von 20 bis 80, bevorzugt 40 bis 70°C oder mit einem Hochdruckhomogenisator wie einem APV Gaulin bzw. mit einem Höchstdruckhomogenisator wie dem Microfluidizer im Druckbereich von 700 bis 1000 bar. Die damit erreichbaren mittleren Durchmesser der wäßrig-dispersen Phase sind kleiner 100 µm, bevorzugt kleiner 10 µm, insbesondere kleiner 1 µm.

Die nanopartikulären, schutzkolloidstabilisierten Carotinoide in der dispersen Phase gewährleisten außerdem, daß diese flüssige Formulierung die gleiche Bioverfügbarkeit und chemische Stabilität aufweist wie die Trockenpulver gemäß EP-B-0 065 193.

Die so hergestellten, erfindungsgemäßen Flüssigformulierungen carotinoidhaltiger Wirkstoffe können zu Färbezwecken in der Aquakultur direkt oder in der Regel nach Verdünnen mit Ölen auf das bereits fertig extrudierte Fischfutter aufgesprüht werden.

Im Lebensmittelbereich lassen sich die flüssigen Carotinoid-Formulierungen zum Färben von u.a. Margarine, Backwaren, Nudeln, Desertprodukte etc. einsetzen, wobei die erfindungsgemäßen W/O-Emulsionen besonders gut geeignet sind, wenn für das Färbeverfahren eine Öllöslichkeit der Formulierung erforderlich ist. Aus anwendungstechnischer Sicht haben diese Flüssigformulierungen außerdem den Vorteil, daß sie einfacher dosierbar sind als vergleichbare Carotinoid-Trockenpulver.

Entsprechendes gilt für die Anwendung der erfindungsgemäßen Carotinoid-Flüssigformulierungen zu Färbezwecken im Kosmetik- und Pharmabereich.

In den nachfolgenden Beispielen wird die Durchführung des erfindungsgemäßen Verfahrens näher erläutert.

### Beispiel 1

85 g einer Astaxanthin-Dispersion mit einem mittleren Teilchendurchmesser der Astaxanthin-Partikel von ungefähr 100 nm, die neben 6 Gew.-% Astaxanthin, 55 Gew.-% Wasser, 20 Gew.-% Saccharose, 15 Gew.-% Gelatine, 2 Gew.-% Ethoxyquin, 1 Gew.-% Konservierungsstoffe sowie 1 Gew.-% Ascorbylpalmitat enthält, wurden nach dem Mischkammermikronisierverfahren gemäß EP-B-0 065 193 hergestellt und anschließend bei 50°C 5 Minuten lang in ein Gemisch aus 300 g Sojaöl und 30 g Polyglycerol Polyricinoleat emulgiert. Die resultierende stabile Emulsion hatte einen Wirkstoffgehalt von ca. 1,2 Gew.-%.

Bei Verwendung einer Zahnkranzdispergiermaschine (Ultraturax® ) als Homogenisator wurden mittlere Tropfendurchmesser der dispersen Phase von ca. 2 µm erreicht. Bei zusätzlicher Anwendung eines Hochdruckhomogenisators (Mikrofluidizer) bei 1000 bar konnten mittlere Tropfendurchmesser von ca. 1 µm erzielt werden.

### Beispiel 2

193 g einer Canthaxanthin-Dispersion mit einem mittleren Teilchendurchmesser der Canthaxanthin-Partikel von ungefähr 150 nm, die neben 4 Gew.-% Canthaxanthin, 70 Gew.-% Wasser, 14 Gew.-% Saccharose, 9 Gew.-% Gelatine, 1 Gew.-% Ethoxyquin, 1 Gew.-% Konservierungsstoffe sowie 1 Gew.-% Ascorbylpalmitat enthält, wurden nach dem Mischkammermikronisierverfahren gemäß EP-B-0 065 193 hergestellt und anschließend bei 60°C 2 Minuten lang in ein Gemisch aus 400 g Sojaöl und 40 g Polyglycerol Polyricinoleat mit Hilfe eines Ultraturax® bei 11000 U/min emulgiert. Zur Verbesserung der Feinverteilung wurde die Emulsion zusätzlich bei ca. 26°C in zwei Passagen in einem Höchstdruckhomogenisator (Mikrofluidizer) bei 900 bar homogenisiert. Die resultierende stabile Emulsion hatte einen Wirkstoffgehalt von ca. 1,2 Gew.-%. Die mittlere Tröpfengröße betrug 0,85 µm.

## Patentansprüche

1. Flüssige, mit Öl mischbare Carotinoid-Zubereitungen, dadurch gekennzeichnet, daß sie als doppelte Dispersionssysteme eine wäßrig-disperse Phase mit einem Partikeldurchmesser kleiner 100 µm, in der schutzkolloid-stabilisierte Teilchen eines oder mehrerer Carotinoide dispergiert vorliegen, in einem Öl als Dispersionsmittel enthalten.

2. Flüssige, mit Öl mischbare Carotinoid-Zubereitungen nach Anspruch 1 dadurch gekennzeichnet, daß die wäßrig-disperse Phase schutzkolloid-stabilisierte Teilchen eines oder mehrerer Carotinoide in öliger, amorpher und/oder kristalliner Form enthält.

3. Flüssige, mit Öl mischbare Carotinoid-Zubereitungen nach den Ansprüchen 1 und 2 dadurch gekennzeichnet, daß sie einen Emulgator mit einem HLB-Wert kleiner 10 enthalten.

4. Flüssige, mit Öl mischbare Carotinoid-Zubereitungen nach den Ansprüchen 1 bis 3 dadurch gekennzeichnet, daß die wäßrig-disperse Phase weitere Zusatzstoffe wie Emulgatoren, Antioxidantien und/oder Konservierungsstoffe enthält.

5. Flüssige, mit Öl mischbare Carotinoid-Zubereitungen nach den Ansprüchen 1 bis 4 dadurch gekennzeichnet, daß der Partikeldurchmesser der wäßrig-dispersen, Carotinoid-haltigen Phase kleiner 10 µm ist.

6. Flüssige, mit Öl mischbare Carotinoid-Zubereitungen nach den Ansprüchen 1 bis 5 dadurch gekennzeichnet, daß sie einen Carotinoidgehalt von 0,01 bis 10 Gew.% aufweisen.

7. Verfahren zur Herstellung von flüssigen, mit Öl mischbaren Carotinoid-Zubereitungen nach den Ansprüchen 1 bis 6 dadurch gekennzeichnet, daß man eine wäßrige, schutzkolloidhaltige Dispersion eines oder mehrerer Carotinoide in Gegenwart eines Emulgators in Öl emulgiert.

8. Verwendung der flüssigen, mit Öl mischbaren Carotinoid-Zubereitungen nach den Ansprüchen 1 bis 6 als Zusatz zu Tierfuttermitteln, Lebensmitteln, Kosmetika und/oder Pharmazeutika.

## Claims

1. A liquid, oil-miscible carotenoid preparation which, as a double dispersion system, comprises an aqueous-disperse phase which has a particle diameter of less than 100 µm and in which particles, stabilized by protective colloid, of one or more carotenoids are present in disperse form, in an oil as a dispersion medium.

2. The liquid, oil-miscible carotenoid preparation as claimed in claim 1, wherein the aqueous-disperse phase comprises particles, stabilized by protective colloid, of one or more carotenoids in oily, amorphous and/or crystalline form.

3. The liquid, oil-miscible carotenoid preparation as claimed in claims 1 and 2, which comprises an emulsifier with an HLB value below 10.

4. The liquid, oil-miscible carotenoid preparation as claimed in claims 1 to 3, wherein the aqueous-disperse phase comprises further additives such as emulsifiers, antioxidants and/or preservatives.

5. The liquid, oil-miscible carotenoid preparation as claimed in claims 1 to 4, wherein the particle diameter of the aqueous-disperse carotenoid-containing phase is less than 10 µm.

6. The liquid, oil-miscible carotenoid preparation as claimed in claims 1 to 5, which has a carotenoid content of from 0.01 to 10 wt.%.

7. A process for preparing the liquid, oil-miscible carotenoid preparation as claimed in claims 1 to 6, wherein an aqueous, protective colloid-containing dispersion of one or more carotenoids is emulsified in oil in the presence of an emulsifier.

8. The use of the liquid, oil-miscible carotenoid preparation as claimed in claims 1 to 6 as an additive for animal feeds, foods, cosmetics and/or pharmaceuticals.

## Revendications

1. Compositions liquides, miscibles à l'huile, de caroténoïdes, caractérisées par le fait qu'elles contiennent en tant que système à double dispersion une phase en dispersion aqueuse à un diamètre de particule inférieur à 100 µm, dans laquelle des particules d'un ou plusieurs caroténoïdes, stabilisées par un colloïde protecteur, sont en dispersion, dans une huile qui sert de milieu de dispersion.

2. Compositions liquides, miscibles à l'huile, de caroténoïdes selon la revendication 1, caractérisées par le fait que la phase en dispersion aqueuse contient des particules, stabilisées par un colloïde protecteur, d'un ou plusieurs caroténoïdes à l'état huileux, amorphe et/ou cristallisé.

3. Compositions liquides, miscibles à l'huile, de caroténoïdes selon les revendications 1 et 2, caractérisées par le fait qu'elles contiennent un agent émulsionnant présentant une valeur HLB inférieure à 10.

4. Compositions liquides, miscibles à l'huile, de caroténoïdes selon les revendications 1 à 3, caractérisées par le fait que la phase en dispersion aqueuse contient d'autres additifs tels que des agents émulsionnants, des antioxydants et/ou des conservateurs.

5. Compositions liquides, miscibles à l'huile, de caroténoïdes selon les revendications 1 à 4, caractérisées par le fait que le diamètre de particule de la phase en dispersion aqueuse contenant les caroténoïdes est inférieur à 10 µm.

6. Compositions liquides, miscibles à l'huile, de caroténoïdes selon les revendications 1 à 5, caractérisées par le fait que leur teneur en caroténoïdes est de 0,01 à 10 % en poids.

7. Procédé pour la préparation de compositions liquides, miscibles à l'huile, de caroténoïdes selon les revendications 1 à 6, caractérisé par le fait que l'on émulsionne une dispersion aqueuse, contenant un colloïde protecteur, d'un ou plusieurs caroténoïdes, en présence d'un agent émulsionnant, dans une huile.

8. Utilisation des compositions liquides, miscibles à l'huile, de caroténoïdes selon les revendications 1 à 6 en tant qu'additifs à des aliments pour animaux, des produits alimentaires, des produits cosmétiques et/ou des produits pharmaceutiques.
